# EUROPEAN PATENT APPLICATION

(11) **EP 2 465 343 A1**
(43) Date of publication of application: **20.06.2012**
(21) Application number: 10195357.8
(22) Date of filing: 16.12.2010
(51) Int. Cl.: A01H 5/02, C12N 15/82, A01H 1/04

(54) **Cultivated plant having tobacco streak virus resistance**

(71) Applicant: Syngenta Participations AG, 4058 Basel (CH)
(72) Inventor: Kulkarni, Naveenkumar, 431105 Mulani Wadgaon Farm (IN)
(74) Representative: Scharrenberg, Christian

(57) **Abstract**

The present invention relates to a cultivated plant, preferably a cultivated sunflower plant comprising a gene (*TSV1*) conferring resistance to tobacco streak virus disease. A method of producing oil and/or meal from sunflower comprising the gene *TSV1* is also provided.

## Description

### FIELD OF INVENTION

The present invention relates to a cultivated plant, preferably a sunflower plant comprising a gene from a wild source which confers tobacco streak virus resistance, obtaining tobacco streak virus resistant plants as well as to the seeds and plant parts derived from said plants. Moreover, the invention relates to a method for obtaining oil and/or meal from seeds of said plants.

### BACKGROUND

Sunflower (*Helianthus annuus*) is a member of *Asteraceae* and is a major oilseed crop in countries such as India and Australia. It is a major source of vegetable oil and is used as cooking oil, in salads and margarine. The meal prepared from seeds after the oil has been extracted can be used for feeding livestock.

Although there have been more than 30 diseases reported in sunflowers, only a few of them are of common occurrence. These include Alternaria blight, rust and downy mildew. Among these, the downy mildew problem has been solved to a great extent by chemical seed treatment (Shirshikar, S.P., 2005, Helia 26(39): 109-116).

A relatively new threat to commercial sunflower production has now emerged in the form of Tobacco Streak Virus (TSV). In India, the disease was noticed for the first time in 1997 at Bagepally (Kolar) near Bangalore (Singh, S.J., et al 1997, Annual Meeting of the Indian Phyto Pathological Society (IPS) West-zone meet on economically important diseases of crop plants, Bangalore, December 18-20, 1997. pp. 24) with the incidence ranging from 10-80 per cent in both open pollinated and hybrid varieties. In subsequent years, outbreaks of this disease in major Indian sunflower-growing states, especially Andhra, Karnataka and Maharashtra, has been a major threat to sunflower cultivation, causing 50 - 80% yield loss. TSV infection of sunflower has also been reported in Australia (Brunt AA et al 1996: Viruses of plants. CAB International, Wellingford).

Once it infects the plant, TSV reproduces and causes the death of tissue in the vicinity of the infection. It spreads along the vascular (nutrient conducting) tissue, leading to wilting and death of other plant parts including leaves and seed heads. The extent of plant damage depends on the growth stage of the plant at the time of infection. If plants are infected as seedlings, the whole plant may be killed. If infection occurs in the mid stages of plant growth, infection may result in death of leaves and deformation and reduction in seed head size. Infection late in the plant lifecycle may result in only minor visual symptoms, with little effect on plant growth. The level of infection within a sunflower crop can vary. In some cases, only a small proportion of plants are affected (<1%) while in other cases a high proportion of plants can be affected, in patches or scattered throughout the field, resulting in significant levels of yield loss (>50%).

Existing practices to control the virus include seed treatment with a residual systemic insecticide. This can provide about three weeks protection to the plant during what is believed to be the most susceptible stage. Good farm hygiene which includes the control of weeds along fence lines, in the crop and in pasture areas is recommended. Planting quick growing barrier crops such as forage sorghum, around commercial sunflower crops may decrease the movement of the vector thrips. Also, where possible the planting of sunflowers close to weedy areas (including weedy pastures) that may act as a host of the virus should be avoided.

TSV is transmitted by thrips in the presence of infected pollen grains (Harvir Singh, 2005, J. Oilseeds Res. 22(1): 90-92). A number of thrips species are known vectors of TSV. They include: *Frankliniella schultzei* (tomato thrips), *Megalurothrips usitatus* (bean blossom thrips), *Scirtothrips doralis* (strawberry thrips), *Thrips parvispinus* (Taiwanese thrips), *Thrips tabaci* (onion thrips), *Frankliniella occidentalis* (Western flower thrips) and *Microcephalothrips abdominalis* (composite thrips).

TSV relies on living plant tissue or pollen to survive. It cannot survive in the soil, or on machinery, and has a very short life outside living susceptible host plant material. It is difficult to combat the disease through single approach due to its viral origin.

Most of the sunflower hybrids currently under cultivation in India have shown various degrees of susceptibility to TSV. Sunflower crop sowing in post rainy season (September onwards) was found to be beneficial for minimizing necrosis incidence (Shirshikar, S.P., 2003, Helia 26(39): 109-116).

Because it cannot survive for long outside a living plant, the infection of crops by TSV relies on the virus surviving in living plants (other crops or weeds) during periods when the crop is not present. Other crops that are known to be susceptible to TSV (and therefore may act as a host) include chickpeas, cotton, mungbeans, peanuts and soybeans. TSV is also known to infect a wide range of weeds such as parthenium weed, black pigweed, blackberry nightshade, green amaranth, and common thornapple.

There is a clear need to find sources of stable TSV resistance which can be introduced into cultivated sunflower lines suitable for the commercial market.

### SUMMARY OF THE INVENTION

The present invention addresses the unfulfilled need for new sources of Tobacco Streak Virus (TSV) resistance. In a first aspect, the invention relates to a cultivated plant, preferably a cultivated sunflower plant comprising a gene, *TSV1,* which confers resistance to tobacco streak virus disease. The new gene was surprisingly found in the wild accession *Helianthus annuus* var ANN2121 (USDA) and successfully transferred to Syngenta cultivated inbred line *H. annuus* var. 0GI1100, representative seed of which has been deposited under accession number NCIMB 41748. The *TSV1* gene is located at the chromosomal locus of the gene conferring resistance to TSV in *H. annuus* var 0GI1100 and also in hybrid line S-293, the representative seed of which has been deposited under accession number NCIMB 41747.

The *TSV1* gene is derivable from *H. annuus* var 0GI1100. There is also provided a cultivated plant, preferably a cultivated sunflower plant, comprising a *TSV1* gene according to the invention and in which plant said gene can be detected by crossing said cultivated plant with a plant of line *H. annuus* var 0GI1100 wherein 100% of F1 plants resulting from said cross are resistant to TSV and a) 100% of said F1 plants produce 100% of F2 progeny plants resistant to TSV, when said cultivated plant is homozygous for the *TSV1* gene; or b) 50% of said F1 plants produce 100% of F2 progeny plants resistant to TSV and 50% of said F1 plants a 3:1 ratio of F2 progeny plants resistant to TSV to F2 progeny plants susceptible to TSV, when said cultivated plant is heterozygous for the *TSV1* gene.

In a second aspect, the invention relates to a method of producing sunflower oil and/or meal comprising the steps:
a) growing and harvesting a cultivated sunflower plant comprising the *TSV1* gene according to the first aspect of the invention;
b) processing seed obtained from the harvested sunflower plant to produce oil and/or meal.

In one embodiment of the method, the harvested sunflower plant of step a) does not exhibit any of the following disease symptoms: mosaicing, general necrosis, stem necrosis, yellowing, stunting and/or head necrosis at the seed setting stage.

The cultivated sunflower plant of step a) can be an inbred line, a hybrid or a dihaploid. In one embodiment the cultivated sunflower plant of step a) is hybrid line S-293 or a descendent thereof. In one embodiment the sunflower plant of step a) is *H. annuus* var 0GI1100 or a descendent thereof.

In a third aspect, the invention relates to oil and/or meal obtained from a cultivated plant of the invention, for example by a method of the second aspect as described herein.

In a fourth aspect, the invention relates to a food product comprising the oil and/or meal of the third aspect.

In a fifth aspect, the invention relates to a method of producing seed from a TSV resistant cultivated sunflower plant containing the gene *TSV1* according to the first aspect of the invention, said method comprising growing, harvesting and obtaining the seed.

In a sixth aspect, the invention relates to a method of reducing TSV infestation in a cultivated sunflower plant comprising use of the gene *TSV1* according to the first aspect of the invention.

In a seventh aspect, the invention relates to a method of enhancing resistance to TSV in a cultivated sunflower plant comprising use of the gene *TSV1* according to the first aspect of the invention.

In an eighth aspect, the invention relates to a method of producing sunflower oil and/or meal comprising the step of processing seed obtained from a cultivated sunflower plant containing the *TSV1* gene according to the first aspect of the invention to produce oil and/or meal. In one embodiment of the method, the cultivated sunflower plant does not exhibit any of the following disease symptoms: mosaicing, general necrosis, stem necrosis, yellowing, stunting and/or head necrosis at the seed setting stage. In one embodiment, the cultivated sunflower plant is an inbred line, a hybrid or a dihaploid. In one embodiment, the cultivated sunflower plant is hybrid line S-293, obtainable from seed represented by NCIMB accession number NCIMB 41747, or *H. annuus* var. 0GI1100, obtainable from seed represented by NCIMB accession number NCIMB 41748.

In a ninth aspect, the invention relates to a method of producing seed from a tobacco streak virus resistant cultivated sunflower plant containing the gene according to the first aspect of the invention, comprising obtaining seeds from the cultivated sunflower plant that has been grown and harvested.

In a tenth aspect, the invention relates to oil and/or meal obtained by a method of the eighth or ninth aspect.

In an eleventh aspect, the invention relates to food product comprising oil and/or meal of the tenth aspect.

In a twelfth aspect, the invention relates to oil and/or meal obtained from a cultivated sunflower plant containing the *TSV1* gene according to the first aspect of the invention, or a fragment thereof.

In a thirteenth aspect, the invention relates to oil and/or meal containing the *TSV1* gene according to the first aspect of the invention, or a fragment thereof.

In a fourteenth aspect, the invention relates to food product comprising oil and/or meal of the twelfth or thirteenth aspect.

In a fifteenth aspect, the invention relates to a vector comprising a gene according to the first aspect of the invention.

In a sixteenth aspect, the invention relates to a host cell comprising the vector of the fifteenth aspect of the invention. In one embodiment, the host cell is a microbial cell.

### DEFINITIONS

"Allele" is defined as one of a pair or series of forms of a gene, which are alternative in inheritance because they are situated at the same locus in homologous chromosomes. "Backcrossing" is defined as a process in which a hybrid progeny is repeatedly crossed back to one of the parents. Different recurrent parents may be used in subsequent backcrosses.

As used herein, the term "construct" refers to an artificially assembled or isolated nucleic acid molecule which includes the gene of the invention. A construct may include the gene or genes of the invention, a marker gene (which in some cases can also be the gene of the invention) and suitable regulatory sequences. The inclusion of regulatory sequences in a construct is sometimes optional, for example, such sequences may not be required in situations where the regulatory sequences of a host cell are to be used. The term construct includes vectors but should not be seen as being limited thereto.

"Crossing" is defined as the transfer of pollen from one plant to a different plant. As used herein, the phrases "sexually crossed" and "sexual reproduction" in the context of the presently disclosed subject matter refers to the fusion of gametes to produce progeny (e.g., by fertilization, such as to produce seed by pollination in plants). A "sexual cross" or "Cross-fertilization" is the fertilization of one individual by another (e.g., cross-pollination in plants).

"Cultivated plant" means any plant of the respective species that is commercially grown for its produce. A cultivated plant has been brought into cultivation and has been selectively bred for growing purposes and excludes those wild-type species which comprise the trait being subject of this invention as a natural trait and/or part of their natural genetics. Inbred lines such as H. annuus 0GI1100 and hybrid lines such H. annuus S-293 as described herein are cultivated plant lines. However, a plant of line H. annuus var ANN2121 obtainable from seed deposited at USDA under accession number PI586818 is a wild type source of the *TSV1* gene and is not a cultivated plant for the purposes of the invention herein described. H. annuus var ANN2121 is thus specifically excluded from the scope of the invention.

"Cytoplasmic male sterile (CMS)": A CMS plant does not produce pollen. CMS is maternally inherited in that the male sterile plant is used as a female parent when crossed with pollen from another sunflower line. In order to produce CMS sunflower lines, a maintainer line is crossed with a CMS plant followed by backcrossing to the maintainer until a male sterile plant is developed which is homologous to the maintainer in all other respects.

"Derived/derivable" with respect to a gene is understood to mean a gene which is transferable from a donor plant to a recipient plant eg introduced by crossing. The presence of the gene can be detected in a recipient plant by several methods known to the skilled person, including by the use of an allelic test as described herein.

"Dihaploid" plants are generated by doubling of haploid plants (single chromosome set) (e.g. through anther culture or microspore culture) to give a complete homozygous plant.

"Dominant" gene effect results in a complete phenotypic manifestation in the heterozygous or homozygous state. However, in some cases a gene dosage effect may be observed, which may result in a slightly stronger and more reproducible phenotype at the homozygous state than in the heterozygous state.

A "gene" is a unit of inheritance. Genes are located at fixed loci in chromosomes. A gene can exist in a series of alternative forms called alleles. A gene can control or contribute to a trait, for example resistance to a disease.

"High oil" and "high seed yield" refers to cultivated sunflower plants which have an oil content or grain yield greater than the check variety PAC-8699 i.e. 38% oil content and 603 kg/ha seed yield when grown at the same time and under the same conditions. Line S-293 and 0GI1100 are both examples of a high oil and high seed yield cultivated sunflower plants.

"High virus pressure" is defined as the environmental conditions required to ensure that a susceptible check plant (for example PAC-8699 as described herein) displays at least one symptom of TSV (mosaicing, general necrosis, stem necrosis, yellowing, stunting and/or head necrosis at the seed setting stage) at the seed setting stage whereas hybrid *H. annuus* S-293 does not when grown at the same time and under the same conditions in the field, for example in Kadappa, Andhra Pradesh and, optionally without the artificial introduction of TSV.

"Homozygous" is understood within the scope of the invention to refer to like alleles at one or more corresponding loci on homologous chromosomes.

"Heterozygous" is understood within the scope of the invention to refer to unlike alleles at one or more corresponding loci on homologous chromosomes.

As used herein, the terms "hybrid", "hybrid plant," and "hybrid progeny" refers to an individual produced from genetically different parents (e.g., a genetically heterozygous or mostly heterozygous individual).

"Inbred line" refers to a genetically homozygous or nearly homozygous population. An inbred line, for example, can be derived through several cycles of brother/sister breedings or of selfing. In some embodiments, inbred lines breed true for one or more phenotypic traits of interest. An "inbred", "inbred individual", or "inbred progeny" is an individual sampled from an inbred line and is distinct from a "hybrid" as defined above.

"Isogenic" plants are genetically identical, except that they may differ by the presence or absence of a gene at a locus conferring a trait or heterologous DNA sequence.

A "line" is a group of plants that display less variation between individuals, usually due to several generations of self pollination. A line can also be a group of plants which have been vegetatively propagated from a single parent by, for example, tissue culture or cell culture.

"Locus" refers to a region on a chromosome which generally comprises a gene, e.g. a gene controlling or contributing to a trait.

"Monogenic" is understood to mean being determined by a single locus.

A "plant" is any plant at any stage of development, particularly a seed plant.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, zygotes and embryos at various stages of development.

"Plant material" or "plant material obtainable from a plant" refers to leaves, stems, roots, flowers or flower parts, fruits, pollen, egg cells, zygotes, seeds, cuttings, cell or tissue cultures, or any other part or product of a plant.

A "plant organ" is a distinct and visibly structured and differentiated part of a plant such as a root, stem, leaf, flower bud, or embryo.

"Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

"Polygenic" is understood to mean being determined by more than one locus.

"Progeny" refers to the descendant(s) of a particular cross. Typically, progeny result from breeding of two individuals, although some species (particularly some plants and hermaphroditic animals) can be selfed (i.e., the same plant acts as the donor of both male and female gametes). The descendant(s) can be, for example, of the F1, the F2, or any subsequent generation.

A "recessive" gene manifests itself only when present at homozygous state. "Regeneration" refers to the development of a plant from tissue culture.

"Resistant" or "resistance" to TSV is a biological mechanism based on the genotype of a plant, by which a resistant plant exhibits no symptoms of TSV disease (does not exhibit any of the symptoms mosaicing, general necrosis, stem necrosis, yellowing, stunting and/or head necrosis) at the seed setting stage, as compared to the plants without resistance (susceptible plants), which have one or more of said symptoms at the seed setting stage. The seed setting stage generally occurs after flowering.

"Selfing" refers to the production of seed by self-fertilization or self-pollination; i.e., pollen and ovule are from the same plant.

"Tester" plant is used to characterize genetically a gene or a trait in a plant to be tested. Typically, the plant to be tested is crossed with a "tester" plant and the segregation ratio of the trait in progeny plants of the cross is scored.

"Trait" or "commercially desirable agronomic trait" is a characteristic or phenotype, for example high oil or high seed yield. A trait may be inherited in a dominant or recessive manner. A trait may be monogenic or polygenic, or may also result from the interaction of one or more genes, in some cases with the environment.

"Transfer" or "transferable" with respect to a gene is understood to mean the introduction of a gene or genetic locus from a donor plant to a recipient plant and whose introduction can be detected, for example, by an allelic test as described herein.

*"TSV1"* gene (for resistance to tobacco streak virus disease) is a gene conferring resistance to TSV upon a sunflower plant and which can be found at the chromosomal locus of the gene conferring resistance to TSV in *H. annuus* var 0GI1100 representative seed of which has been deposited under accession number NCIMB 41748. The presence of a *TSV1* gene can be detected in *H. annuus* var 0GI1100, for example, by an allelic test as described herein.

### BRIEF DESCRIPTION OF THE DRAWINGS

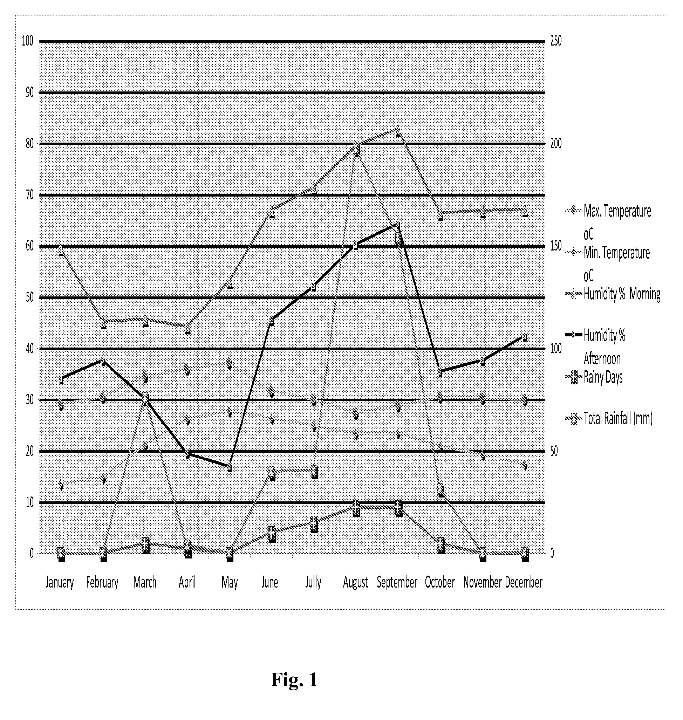

Fig. 1 is a graphical representation of typical weather conditions in which a sunflower plant of the present invention was grown.

### DETAILED DESCRIPTION OF THE INVENTION

The present invention relates to a cultivated plant, preferably a cultivated sunflower plant comprising a gene, *TSV1,* conferring resistance to tobacco streak virus disease (TSV). In one embodiment, the cultivated plant is a sunflower plant selected from the group *Helianthus annus*, *Helianthus tuberosus.* In one embodiment, the cultivated plant is *H*. *annuus.* The *TSV1* gene was surprisingly found in wild accession *H. annuus* ANN2121 (obtained from USDA; accession number P1586818; originally sourced from Montana, USA). The *TSV1* gene has been transferred to Syngenta cultivated line *H. annuus* var 0GI1100 and can be introduced into another sunflower plant by crossing. The *TSV1* gene is located at the chromosomal locus of the gene conferring resistance to TSV in Syngenta cultivated line *H. annuus* var 0GI1100, or in Syngenta hybrid line S-293, representative seed of which has been deposited under accession number NCIMB 41747. The *TSV1* gene is derivable from *H. annuus* var 0GI1100.

There is also provided seed and parts of the cultivated plant of the invention, and methods of making and using such a cultivated plant. There is provided a cultivated sunflower plant comprising gene *TSV1* of the invention conferring upon said plant resistance to TSV. Resistance to TSV may be tested and assessed as described in Example 1. TSV infection of a cultivated sunflower plant of the invention may occur by artificial inoculation, for example by mechanical sap inoculation, or by growing in an area with high virus pressure. However, the person skilled in the art would also be able to use other protocols or methods to determine resistance to the virus.

A cultivated plant of the invention can be homozygous for the *TSV1* gene. In one embodiment, the *TSV1* gene is dominant. In one embodiment, the resistance to TSV is monogenic. Resistance to TSV can be monogenic and dominant. Seed of a representative cultivated sunflower plant comprising the *TSV1* gene according to the present invention, hybrid line S-293, was deposited with NCIMB, Aberdeen AB21 9YA, Scotland, UK under accession number NCIMB 41747 on 6 August 2010. Seed of another representative cultivated sunflower plant comprising the *TSV1* gene according to the present invention, cultivated inbred line 0GI1100, was deposited with NCIMB, Aberdeen AB21 9YA, Scotland, UK under accession number NCIMB 41748 on 6 August 2010. Both lines are homozygous for the *TSV1* gene. In one embodiment, the *TSV1* gene is derivable from *H. annuus* var 0GI1100.

The invention also provides a cultivated plant, preferably a cultivated sunflower plant comprising a gene, *TSV1,* according to the invention. Said gene can be detected by crossing said cultivated sunflower plant with a plant of line *H. annuus* var 0GI1100, wherein 100% of F1 plants resulting from said cross are resistant to TSV and a) 100% of said F1 plants produce 100% of F2 progeny plants resistant to TSV, when said sunflower plant is homozygous for the *TSV1* gene; or b) 50% of said F1 plants produce 100% of F2 progeny plants resistant to TSV and 50% of said F1 plants a 3:1 ratio of F2 progeny plants resistant to TSV to F2 progeny plants susceptible to TSV, when said sunflower is heterozygous for the *TSV1* gene. In one embodiment, the gene, *TSV1* is in homozygous form.

The *TSV1* gene is located at the same chromosomal location in a cultivated sunflower plant as the gene conferring resistance to TSV in *H. annuus* var 0GI1100. A cultivated sunflower plant comprising a *TSV1* gene can be identified and defined by an allelic test as described herein. An allelic test is performed by making a cross between a plant of a "tester" line (i.e. "tester" plant) and the plant to be tested, and determining the segregation ratio of plants resistant to TSV to plants susceptible to TSV in the progeny of the cross. Typically, FI progeny plants and F2 progeny plants resulting from self-pollination of the FI progeny plants are assessed. Progeny plants resulting from backcrosses with the parents and plants obtained after self-pollination of backcross progeny plants may also be assessed. The segregation ratio of resistant to susceptible plants can also be determined in subsequent generations of self- pollination and backcross with the parents. A tester plant is a plant comprising a *TSV1* gene. A tester plant can be a plant of *H. annuus* var 0GI1100. *H. annuus* var 0GI1100 is homozygous for the *TSV1* gene.

If a tester plant homozygous for the *TSV1* gene and a cultivated plant to be tested homozygous for a single gene conferring resistance to TSV are used as parents in a cross, and only resistant plants are recovered in the FI and F2 progenies (i.e. no segregation of resistant and susceptible plants), the plant to be tested comprises a *TSV1* gene, and can be defined as a plant of the invention. Accordingly, in one embodiment, a plant of the invention is a cultivated *H. annuus* plant comprising a *TSV1* gene, wherein when said cultivated *H. annuus* plant is crossed with a plant of line *H. annuus* var 0GI1100, 100% of FI plants resulting from the cross are resistant to TSV, and 100% of the FI plants produce 100% of F2 progeny plants resistant to TSV, when said *H. annuus* plant is homozygous for a *TSV1* gene. In this situation, if FI or F2 progeny plants are backcrossed with the parents and the progeny plants resulting from the backcrosses are self-pollinated, only resistant plants are recovered in the progeny plants resulting from the backcrosses and from subsequent self-pollination.

If a tester plant homozygous for the *TSV1* gene and a cultivated plant to be tested heterozygous for the single gene conferring resistance to TSV are used as parents in a cross, and only resistant plants are recovered in the FI progeny, and 100% of resistant F2 plants are recovered in the progeny of 50% of the FI plants and a 3:1 ratio of resistant to susceptible F2 plants are recovered in the progeny plants of 50% of the FI plants, the plant to be tested comprises a *TSV1* gene, and can be defined as a plant of the invention. Accordingly, in one embodiment, a plant of the invention can be an *H. annuus* plant comprising a *TSV1* gene, wherein when said *H. annuus* plant is crossed with a plant of line *H. annuus* var 0GI1100, 100% of FI plants resulting from said cross are resistant to TSV, and 50% of the FI plants produce 100% of F2 progeny plants resistant to TSV and 50% the FI plants produce a 3:1 ratio of resistant to susceptible F2 plants, when said sunflower plant is heterozygous for a *TSV1* gene.

By contrast, if a tester plant homozygous for the *TSV1* gene and a plant to be tested homozygous for a single gene conferring resistance to TSV are used as parents in a cross, and susceptible plants are found among F2 progeny plants, the plant to be tested contains a TSV resistance gene located at a different locus from that of the *TSV1* gene. The plant to be tested thus does not comprise a *TSV1* gene and is not a plant according to the invention. Susceptible plants are also found among F2 progeny plants if the plant to be tested is heterozygous for a single gene conferring resistance to TSV located at a different locus from that of the *TSV1* gene. In this situation, if FI or F2 progeny plants are backcrossed with the parents and the progeny plants resulting from the backcross are self-pollinated, susceptible plants are found in the progeny obtained after the self-pollination following the backcross.

The person skilled in the art would know how to carry out an allelic test, determine the appropriate number of progeny plants to be tested in each generation, and analyze the results of the test. For example, if the tester plant and the plant to be tested are both homozygous for a dominant single gene conferring resistance to the virus but located at different, unlinked chromosomal loci, one would expect only resistant plants in the FI progeny, but a 15:1 segregation of resistant to susceptible plants in F2 progeny plants. In this case, if the tester plant is homozygous for the *TSV1* gene and the plant to be tested is heterozygous for the single dominant gene located at a different, unlinked chromosomal locus one would expect only resistant plants in the FI progeny, and a 3:1 segregation of resistant to susceptible plants in F2 plants of 50% of the FI plants and a 15:1 segregation of resistant to susceptible plants in F2 plants of 50% of the FI plants.

These segregation ratios may deviate from the above figures if the single dominant gene located at a different, unlinked chromosomal locus confers a milder or less stable resistance, for example moderate resistance to TSV as described herein. In this case, some of the plants comprising the single dominant gene located at a different, unlinked chromosomal locus may be rated as susceptible. If the two chromosomal loci are linked, a higher ratio of resistant to susceptible plants would be expected.

The cultivated plant to be tested in the allelic test can be a cultivated *H. annuus* plant. The cultivated plant to be tested can be an inbred line, a dihaploid or a hybrid line.

It is also understood that in rare cases a cultivated plant comprising a gene conferring resistance to TSV may be rated as susceptible due to aberrant seed germination or abnormal plant development. Such plants are however not considered in the allelic tests and in determining the segregation ratios and are generally eliminated in the disease testing.

Other types of crosses and allelic test may also be carried out by a person skilled in the art. Additional generations may also be evaluated. A tester cross can also be carried out with a tester plant heterozygous for a *TSV1* gene and a plant to be tested heterozygous for a gene to be tested. The person skilled in the art would know how to carry out and interpret the result of these crosses.

Based on the description of the present invention, the skilled person is able to recognize a cultivated sunflower plant of the instant invention, for example by performing an allelic test, for example an allelic test as described herein. Accordingly, in one embodiment, the present invention also further provides a method of identifying a sunflower plant of the instant invention comprising crossing a sunflower plant with a tester plant and determining the segregation ratio of the resistance to TSV in the resulting progeny. Based on the description of the present invention, the skilled person is able to recognize a plant resistant to TSV (also called herein "resistant plant") and a plant susceptible to TSV (also called herein "susceptible" plant), for example using a disease testing method as described herein. In one embodiment, the *TSV1* gene confers high level of resistance to TSV upon a sunflower plant, for example when tested and assayed as described in Example 1 below. Accordingly, in one embodiment, a plant resistant to TSV according to the present invention is a plant resistant to TSV (also called herein "resistant plant"). In one embodiment, the cultivated sunflower plant according to the present invention resistant to TSV is a *H. annuus* plant, for example *H. annuus* var 0GI1100. In one embodiment, a plant of the invention is hybrid line S-293. In one embodiment, a resistant plant according to the present invention is identified and defined by an allelic test, for example an allelic test as described herein.

In the disease testing methods as described herein, a resistant plant shows no symptoms of TSV infection. Based on the description of the present invention, the skilled person is able to recognize a plant of the present invention which is resistant to TSV, for example by using a disease testing method as described herein in the examples section. An example of a *H. annuus* plant resistant to TSV is a plant of hybrid line S-293. A plant which is resistant to TSV shows no symptoms of the disease at the seed setting stage. Accordingly, in one embodiment, a plant resistant to TSV shows an equivalent level of resistance as that of a plant of hybrid line S-293 or *H. annuus* var 0GI1100, for example in a disease test as disclosed herein. In one embodiment, a *H. annuus* plant of the present invention is more resistant to TSV than currently available *H. annuus* plants, in particular when tested in a disease testing as described herein.

The *TSV1* gene was transferred to several varieties of sunflower plants. For example, the *TSV1* gene was transferred to plants of the *H. annuus* var FR818 variety and to plants of the *H. annuus* var RW666 variety. Accordingly, in one embodiment, the present invention further provides a method of transferring a *TSV1* gene to a sunflower plant lacking said gene comprising: a) obtaining a plant comprising a *TSV1* gene; b) crossing it to a plant lacking said gene; c) obtaining plants of the cross of step b); d) selecting a plant of step c) comprising the *TSV1* gene. In one embodiment, the method further comprises: e) backcrossing a plant resulting from step d) with a sunflower plant, and f) selecting for a sunflower plant comprising the *TSV1* gene. In one embodiment, the method further comprises obtaining an inbred sunflower plant comprising the *TSV1* gene, and, in one embodiment, the method further comprises crossing said inbred sunflower plant to another sunflower plant to produce a hybrid sunflower plant comprising the *TSV1* gene. In one embodiment, the plant of step a) comprising a *TSV1* gene is a plant of *H. annuus* var 0GI1100. In one embodiment, the present invention provides a sunflower plant obtainable by any one of the methods as described herein, wherein the plant comprises the *TSV1* gene. In one embodiment, such plant is a sunflower resistant to TSV. In one embodiment, the present invention also provides methods of producing a sunflower plant comprising a *TSV1* gene, for example comprising the steps described herein.

The present invention also provides a method of using a *TSV1* gene to confer resistance to TSV upon a sunflower plant susceptible thereto. In one embodiment, the *TSV1* gene is present in the homozygous state in said sunflower plant. In one embodiment, the method comprises: a) obtaining a plant comprising a *TSV1* gene; b) crossing it to a plant susceptible to TSV; c) obtaining plants of the cross of step b); d) selecting a plant of step c) which is resistant to TSV. In one embodiment, the method further comprises: e) backcrossing a plant resulting from step d) with a sunflower plant, and f) selecting for a sunflower plant, which is resistant to TSV. In one embodiment, the method further comprises obtaining an inbred sunflower plant, which is resistant to TSV, and, in one embodiment, the method further comprises crossing said inbred sunflower plant to another sunflower plant to produce a hybrid sunflower plant, which is resistant to TSV. In one embodiment, the plant of step a) comprising a *TSV1* gene is a plant of *H. annuus* var 0GI1100. The present invention also relates to a cultivated sunflower plant obtainable by any one of the methods as described herein, wherein the plant is resistant to TSV.

Commercial sunflowers are generally FI hybrids produced from the cross of two parental lines (inbreds). The development of hybrids requires, in general, the development of homozygous inbred lines, the crossing of these lines, and the evaluation of the crosses. Pedigree breeding and recurrent selection breeding methods are used to develop inbred lines from breeding populations. Breeders combine the genetic backgrounds from two or more inbred lines or various other germplasm sources into breeding pools from which new inbred lines are developed by selfing and selection of desired phenotypes. The new inbreds are crossed with other inbred lines and the hybrids from these crosses are evaluated to determine which of those have commercial potential. Plant breeding and hybrid development are expensive and time-consuming processes.

Accordingly, in one embodiment, the present invention also provides a method of producing hybrid seed comprising crossing a plant comprising a *TSV1* gene of the invention, wherein said plant is an inbred line, with a plant of another inbred line; and harvesting hybrid seed resulting from the cross. In one embodiment, both inbred lines in the cross are a plant of the present invention. In one embodiment, the present invention also provides hybrid seed produced by a method as described herein, wherein said hybrid seed comprises a *TSV1* gene. In one embodiment, a hybrid seed of the present invention is a seed of hybrid S-293. Pedigree breeding starts with the crossing of two genotypes, each of which may have one or more desirable characteristics that is lacking in the other or which complements the other. If the two original parents do not provide all the desired characteristics, other sources can be included in the breeding population. In the pedigree method, superior plants are selfed and selected in successive generations. In the succeeding generations, the heterozygous condition gives way to homogeneous lines as a result of self-pollination and selection. Typically in the pedigree method of breeding five or more generations of self-pollination and selection is practiced: F1 to F2; F3 to F4; F4 to F5, etc.

A single cross hybrid results from the cross of two inbred lines, each of which has a genotype that complements the genotype of the other. The hybrid progeny of the first generation is designated F1. In the development of commercial hybrids only the F1 hybrid plants are sought. Preferred F1 hybrids can be more vigorous than their inbred parents. This hybrid vigor, or heterosis, can be manifested in many polygenic traits, including increased vegetative growth and high seed yield and high oil content. Sunflower plants can be easily cross-pollinated. A trait is readily transferred from one sunflower plant to another sunflower plant to further obtain commercial lines. The introgression of a trait into a line is for example achieved by recurrent selection breeding, for example by backcrossing. In this case, the line (recurrent parent) is first crossed to a donor inbred (the non-recurrent parent) that carries the trait. The progeny of this cross is then mated back to the recurrent parent followed by selection in the resultant progeny for the trait. After three, preferably four, more preferably five or more generations of backcrosses with the recurrent parent with selection for the trait, the progeny is heterozygous for the locus harboring the resistance, but is like the recurrent parent for most or almost all other genes. Selection for the trait is carried out after each cross.

Accordingly, in one embodiment, a plant of the invention further comprises one or more traits. The traits can be introgressed from another sunflower line, and the resulting plants have essentially all of the morphological and physiological characteristics of plants of the plant of the invention in addition to the one or more introgressed traits. The invention also relates to seeds of the plant of the invention into which one or more traits have been introgressed and to a plant of the invention into which one or more traits have been introgressed. The invention further relates to methods for producing a sunflower plant by crossing a plant of the invention into which one or more traits have been introgressed with themselves or with another sunflower line. The invention also further relates to hybrid sunflower seeds and plants produced by crossing a plant of the invention into which one or more traits have been introgressed with another sunflower line. Examples of traits transferred to a plant of the invention include, but are not limited to, herbicide tolerance, resistance for bacterial, fungal, or viruses additional to TSV, insect resistance, high seed yield and/or high oil content. In one embodiment, a sunflower plant of the present invention is male sterile. Male sterile lines do not produce viable pollen and cannot self-pollinate. By eliminating the pollen of one parental variety in a cross, a plant breeder is assured of obtaining hybrid seed of uniform quality. Therefore, the present invention provides a male sterile sunflower plant, including seeds and materials of said plant and the progeny thereof. In one embodiment, a plant of the invention is a maintainer plant. In one embodiment, a plant of the invention is an inbred, a hybrid, or a dihaploid. In one embodiment, a plant of the invention is produced by pedigree breeding or by recurrent selection breeding. In one embodiment, a plant of the invention has commercially desirable agronomic trait or characteristics such as high oil content and/or high seed yield. In one embodiment, the present invention provides a method of producing seed of a plant of the present invention comprising: a) growing a plant of the present invention; b) allowing said plant to self-pollinate; c) harvesting seeds from said plant.

The present invention also provides a cultivated sunflower plant comprising a *TSV1* gene, wherein when said sunflower plant is crossed with a plant of line *H. annuus* var 0GI1100, 100% of FI plants resulting from said cross are resistant to TSV, and: a) 100% of said F1 plants produce 100% of F2 progeny plants resistant to TSV, when said sunflower plant is homozygous for a *TSV1* gene; or b) 50% of said FI plants produce 100% of F2 progeny plant resistant to TSV and 50% said FI plants produce a 3:1 ratio of F2 progeny plants resistant to TSV to F2 progeny plants susceptible to TSV, when said sunflower plant is heterozygous for a *TSV1* gene. In one embodiment, the *TSV1* gene is derivable from a plant of *H. annuus* var 0GI1100. In one embodiment, the cultivated plant is homozygous for said *TSV1* gene. In one embodiment, the cultivated plant is an inbred line, a hybrid or a dihaploid. In one embodiment, the plant is male-sterile.

The present invention further provides a sunflower plant resistant to TSV, wherein the plant comprises a *TSV1* gene as described herein. In one embodiment, the plant is an *H. annuus* plant of the invention. In one embodiment, the plant is hybrid line S-293, representative seed of which has been deposited under accession number NCIMB 41747. In one embodiment, the plant is *H. annuus* var 0GI1100. In one embodiment, the *TSV1* gene is derivable from a plant of *H. annuus* var 0GI1100. In one embodiment, the plant is homozygous for said *TSV1* gene. In one embodiment, the plant is an inbred line, a hybrid or a dihaploid. In one embodiment, the plant is male-sterile. In one embodiment, the plant can be identified in an allelic test, for example by an allelic test as described herein.

The present invention further provides seed or part of any one of the sunflower plants as described herein. In one embodiment, the plant part is a cell, pollen, or ovule. The present invention further provides a method of producing seed of a sunflower plant comprising: growing any one of the cultivated sunflower plants comprising the *TSV1* gene as described herein; allowing said sunflower plant to self-pollinate; and harvesting seeds from said sunflower plant.

The present invention further provides a method for producing a sunflower plant comprising a *TSV1* gene comprising: crossing any one of the sunflower plants comprising a *TSV1* gene as described herein and a sunflower plant lacking a *TSV1* gene; obtaining a progeny plant from the cross; and selecting in the progeny a sunflower plant comprising a *TSV1* gene. The present invention further provides a sunflower plant comprising a *TSV1* gene obtainable by the method. The present invention further provides a method of transferring a *TSV1* gene to a sunflower plant lacking said gene comprising: crossing any sunflower plant comprising a *TSV1* gene as described herein and a sunflower plant lacking a *TSV1* gene; obtaining progeny plants from the cross; and selecting in said progeny a sunflower plant comprising a *TSV1* gene. The present invention further provides a sunflower plant comprising a *TSV1* gene obtainable by a method as described herein.

The present invention further provides a method of conferring resistance to TSV upon a sunflower plant comprising: crossing any one of the sunflower plants comprising a *TSV1* gene as described herein and a TSV susceptible sunflower plant not comprising a *TSV1* gene; obtaining a progeny plant from the cross; and selecting in said progeny a sunflower plant resistant to TSV. The present invention further provides a sunflower plant resistant to TSV obtainable by the method as described herein. The present invention further provides a method for producing hybrid seed comprising: crossing any one of plants with a *TSV1* gene as described herein, wherein said plant is an inbred line, with a plant of another inbred line; and harvesting hybrid seed resulting from the cross. The present invention further provides hybrid seed produced by the method as described herein, wherein said hybrid seed comprises a *TSV1* gene as described herein.

The present invention further provides a method for producing a sunflower plant comprising a *TSV1* gene as described herein comprising: selecting and crossing a sunflower plant comprising a *TSV1* gene, for example *H. annuus* var 0GI1100 with a sunflower plant lacking a *TSV1* gene; rescuing an embryo resulting from the cross; regenerating a plant from said embryo; and selecting a plant that is resistant to TSV. The present invention further provides a sunflower plant obtainable by the method as described herein, wherein said plant comprises a *TSV1* gene. In one embodiment, the method comprises growing a tissue culture of regenerable cells of the sunflower plant resistant to TSV under conditions suitable for regenerating the sunflower plant. The present invention also relates to a plant or plant part or a seed, produced from the regenerated sunflower plant. A method of producing a sunflower plant according to the invention resistant to TSV may also include planting a susceptible check sunflower plant and growing it at the same time as the resistant sunflower plant and under the same conditions. The susceptible check plant displays at least one of mosaicing, general necrosis, stem necrosis, yellowing, stunting and head necrosis at the seed setting stage. In one embodiment, the susceptible check displays mosaicing at the seed setting stage. An example of a susceptible check plant is line PAC-8699 (from Advanta India Limited).

There is also provided a method of detecting the *TSV1* gene according to the invention in a cultivated sunflower plant comprising crossing a cultivated sunflower plant with a plant of line *H. annuus* var 0GI1100, wherein 100% of F1 plants resulting from said cross are resistant to TSV and a) 100% of said F1 plants produce 100% of F2 progeny plants resistant to TSV, when said sunflower plant is homozygous for the *TSV1* gene; or b) 50% of said F1 plants produce 100% of F2 progeny plants resistant to TSV and 50% of said F1 plants a 3:1 ratio of F2 progeny plants resistant to TSV to F2 progeny plants susceptible to TSV, when said sunflower is heterozygous for the *TSV1* gene.

The invention further relates to a method of producing sunflower oil and/or meal comprising the steps:
a) growing and harvesting a cultivated sunflower plant comprising the *TSV1* gene according to the invention;
b) processing seed obtained from the harvested sunflower plant to produce oil and/or meal.

In one embodiment of the method, the harvested sunflower plant of step a) does not exhibit any of the disease symptoms mosaicing, general necrosis, stem necrosis, yellowing, stunting and/or head necrosis at the seed setting stage.

The cultivated sunflower plant comprising the *TSV1* gene according to the present invention can be an inbred, a hybrid or a dihaploid. In one embodiment, the plant is an *H. annuus* plant of the invention. In one embodiment, the plant is hybrid line S-293. In one embodiment, the cultivated plant is *H. annuus* var 0GI1100. In one embodiment, the resistance to TSV is conferred by the *TSV1* gene as described herein. In one embodiment, a cultivated plant comprising a *TSV1* gene is identified and defined by an allelic test, for example by an allelic test as described herein. In one embodiment, a cultivated plant of the invention is homozygous for the *TSV1* gene. In one embodiment, the *TSV1* gene is dominant. In one embodiment, the resistance to TSV is monogenic. In one embodiment, the resistance to TSV is monogenic and dominant. In one embodiment, the cultivated sunflower plant comprising a *TSV1* gene is obtainable by one of the methods as described herein. In one embodiment, the plant comprises at least one commercially desirable agronomic trait.

The present invention also relates to oil and/or meal obtained by a method of the invention. The method of preparing oil of seed comprises crushing seeds and separating out the oil from the meal. The seeds of the cultivated sunflower plant of the invention resistant to TSV are collected for this purpose. The oil of seed and meal of seed thus obtained is further processed to make it suitable for use in food products. Further processing of oil of seed includes refining, bleaching, winterizing or deodorizing. In a preferred embodiment, further processing of oil of seed includes refining, bleaching and deodorizing. The meal of seed thus prepared can also be fed to livestock.

The present invention also relates to a method of producing seed from a tobacco streak virus resistant cultivated sunflower plant comprising growing, harvesting said plant and obtaining the seed. In one embodiment, the cultivated sunflower plant comprises a *TSV1* gene according to the present invention. In one embodiment, the cultivated plant is an inbred, a hybrid or a dihaploid. For example, the cultivated plant can be an *H. annuus* plant of the invention such as hybrid line S-293 or *H. annuus* var 0GI1100. In one embodiment, the resistance to TSV is conferred by the *TSV1* gene as described herein. In one embodiment, a cultivated plant comprising a *TSV1* gene is identified and defined by an allelic test, for example by an allelic test as described herein. In one embodiment, a cultivated plant of the invention is homozygous for the *TSV1* gene. In one embodiment, the *TSV1* gene is dominant. In one embodiment, the resistance to TSV is monogenic. In one embodiment, the resistance to TSV is monogenic and dominant. In one embodiment, the cultivated sunflower plant comprising a *TSV1* gene is obtainable by one of the methods as described herein. In one embodiment, the plant comprises at least one commercially beneficial agronomic trait.

The present invention also relates to a method of reducing tobacco streak virus infestation in a cultivated plant, for example a cultivated sunflower plant, comprising use of the gene *TSV1* according to the invention. In one embodiment, the cultivated plant is an inbred, a hybrid or a dihaploid. For example, the cultivated plant can be an *H. annuus* plant of the invention such as hybrid line S-293 or *H. annuus* var 0GI1100. In one embodiment, the reduced infestation of TSV is due to activity of the *TSV1* gene as described herein. In one embodiment, a cultivated plant comprising a *TSV1* gene and with reduced TSV infestation can be identified and defined by an allelic test, for example by an allelic test as described herein. In one embodiment, a cultivated plant of the invention with reduced TSV infestation is homozygous for the *TSV1* gene. In one embodiment, the *TSV1* gene is dominant. In one embodiment, the resistance to TSV is monogenic. In one embodiment, the resistance to TSV is monogenic and dominant. In one embodiment, the cultivated sunflower plant comprising a *TSV1* gene is obtainable by one of the methods as described herein.

The present invention also relates to a method of enhancing resistance to tobacco streak virus in a cultivated plant, for example a cultivated sunflower plant, comprising use of the *TSV1* gene according to the invention. In one embodiment, the cultivated plant is an inbred, a hybrid or a dihaploid. For example, the cultivated plant can be an *H. annuus* plant of the invention, such as hybrid line S-293 or *H. annuus* var 0GI1100. In one embodiment, the resistance to TSV is conferred by the *TSV1* gene as described herein. In one embodiment, a cultivated plant comprising a *TSV1* gene is identified and defined by an allelic test, for example by an allelic test as described herein. In one embodiment, a cultivated plant of the invention is homozygous for the *TSV1* gene. In one embodiment, the *TSV1* gene is dominant. In one embodiment, the resistance to TSV is monogenic. In one embodiment, the resistance to TSV is monogenic and dominant. In one embodiment, the cultivated sunflower plant comprising a *TSV1* gene is obtainable by one of the methods as described herein.

A plant according to the invention showing resistance to TSV does not display any of the following disease symptoms at the seed setting stage or, in one embodiment, at 60 days after sowing (DAS):
a) Mosaicing (intermingled patches of light and dark green on leaves due to chlorophyll destruction by virus)
b) General necrosis (death of plant tissue on leaf/stem/leaf petiole and localised death of cells)
c) Stem necrosis (necrotic patches seen on stem. The plant will often topple over at the necrosis point)
d) Head necrosis (sepals dry out due to necrosis resulting in poor or no grain filling)
e) Yellowing (entire plant turns yellow due to destruction of chlorophyll)
f) Stunting (reduction in height due to reduction of internodal distance)

For production of a hybrid sunflower plant, either one of the parent sunflower plants may comprise a genetic determinant encoding cytoplasmic male sterility whilst the other parent sunflower plant comprises a genetic determinant encoding fertility restoration. Either one of these plants may also have at least one commercially desirable agronomic trait such as high seed volume, high seed yield, high oil content and so forth. Hybrid sunflower plants also have the trait of TSV resistance. It was found that F1 progeny produced by crossing a cultivated 0GI1100 plant with Cytoplasmic Male Sterile plants (CMS) had very good resistance even after 3-4 rounds of artificial inoculation.

TSV resistant *H. annuus* var. 0GI1100 progeny show the following physical characteristics: Tall, branching, dark green broad leaves with coarse serration, light yellow flower, flat head, lateral head as described herein to central head with curved stem and black non-striped seeds.

The *TSV1* gene of the invention may be part of a construct. The construct may be a vector. The vector may be a plasmid vector, a viral vector, or any other suitable vehicle adapted for the insertion of foreign sequences and introduction into eukaryotic cells. In one embodiment, the vector is a plasmid vector, such as an *Agrobacterium* vector. In another embodiment, the vector is a viral vector, such as a Epstein-Barr virus-, bovine papilloma virus-, adenovirus- and adeno-associated virus-based vector.

The vector may be an expression vector capable of directing the expression of the *TSV1* gene of the invention in a particular host cell or organism. In another embodiment, the vector is a cloning vector, such as a binary vector.

The construct may further include a marker gene and suitable regulatory sequences. Exemplary marker genes include genes that confer nuclear drug resistance (such as kanamycin resistance), genes that confer antibiotic resistance (such as hygromycin or bialaphos resistance), or genes that confer herbicide resistance (such as sulfonylurea, phosphinothricin or glyphosate resistance). Suitable regulatory sequences include 5' regulatory sequences (such as promoters and translational regulatory sequences) and 3' regulatory sequences (such as terminators). Exemplary promoters include the Cauliflower Mosaic Virus (CaMV) 35S promoter, the figwort mosaic virus 35S promoter, the T-DNA mannopine synthetase promoter, the nopaline synthase (NOS) promoter and the octopine synthase (OCS) promoter. Such regulatory sequences may not be required in situations where the regulatory sequences of a host cell are to be used.

The host cell may be a prokaryotic cell such as a microbial cell (for example *E. coli* or *Agrobacterium*), or an eukaryotic cell such as a yeast cell, an insect cell, an amphibian cell or a mammalian cell. In one embodiment, the host cell is a microbial cell. In another embodiment, the host cell is a plant cell. The host cell may be a cell that is suitable for growing in culture under laboratory conditions. In one embodiment, the host cell differs or is modified biologically and physiologically from any cell naturally occurring in a plant or animal.

### SEED DEPOSIT DETAILS

The following seed samples were deposited with NCIMB, Ferguson Building, Craibstone Estate, Bucksburn, Aberdeen AB21 9YA, Scotland, UK, on 6 August 2010 under the provisions of the Budapest Treaty in the name of Syngenta Participations AG:
NCIMB 41748 *Helianthus annuus* var. 0GI1100.
NCIMB 41747 *Helianthus annuus* hybrid S-293.

### EXAMPLE 1

### Artificial inoculation and methodology for identifying TSV resistance in sunflower

*Maintenance of TSV culture* Tobacco streak virus culture was typically maintained on TSV susceptible hybrid sunflower PAC-8699 (Advanta India Limited, Andhra Pradesh, India) and cowpea (*Vigna unguiculata* var Shifali). TSV infected sunflower leaves were collected from the field. Presence of virus was confirmed by ELISA using protocol AS0615TSV sunflower (DSMZ, Germany). The virus was inoculated on fully expanded 6-7 days old cowpea seedlings with inoculation buffer (0.05M Potassium Phosphate Buffer (pH 7.1) incorporated with 2-mercaptoethanol). The mechanical sap inoculation procedure involved grinding one part of TSV infected cowpea leaf tissue in 9 parts of chilled inoculation buffer. A small pinch of abrasive, such as Celite powder was added, mixed well and then applied to fully expanded leaves of sunflower seedlings. The leaves were allowed to dry. The leaves were washed with distilled water. The inoculated plants were then incubated under polyhouse conditions at 27°C. Brown colored circular rings were observed on inoculated cowpea leaves 4-5 days after inoculation. These leaves served as a source of virus inoculum and used for artificial inoculation of either parthenium or sunflower. Parthenium were also inoculated with TSV so as to maintain high viral pressure in the field.

*Collection and inoculation of Parthenium hysterophorus L.* Parthenium seedlings at the two to three leaf stage were collected and transplanted to a poly bag containing soil. The seedlings were watered regularly to enable good establishment. The established seedlings were artificially inoculated with TSV from cowpea leaves using the mechanical sap inoculation technique with inoculation buffer.

*Transplanting of Parthenium* The inoculated Parthenium seedlings were transplanted 1.5 feet apart in the field in accordance with the following layout:
Two rows all around the border of the field.
Two rows after every four sunflower strips.
One row after every five rows of sunflower in a strip.
8-10 plants planted in each row

*Sowing of sunflower* The sunflowers were sown once the transplanted Parthenium had started flowering. The susceptible check (sunflower line 8699) was sown after every two rows of sunflower.

*Crop Management* Normal sunflower agronomy was practiced for good crop production. At the time of sowing 50% of nitrogen, 100% of potash and potassium (P&K) were applied as basal dose. If any gaps were observed, these were filled on the 8th or 9th day after sowing (DAS). Thinning was performed on the 18th or 22nd DAS. Irrigation and weeding was done as and when needed. Two inter-cultivations were carried with 25% of nitrogen; the first one after the final thinning, and the second one between 35-40 DAS. The parthenium were uprooted between the 55th - 60th DAS of sunflower. Insecticides were applied for the management of head borer and defoliators. Two sprays of boron were applied, one at button stage of the head and the other at the flower opening stage. Three sprays of foliar nutrients were applied; one at the vegetative stage, the remaining two at the flowering stage.

The TSV screening block for sunflower was laid out in the following manner:

| Row No | Strip 1 |
|---|---|
| 1 | Parthenium |
| 2 | Parthenium |
| 3 | Entry 1 |
| 4 | Entry 1 |
| 5 | Susceptible check |
| 6 | Entry 2 |
| 7 | Entry 2 |
| 8 | Parthenium |
| 9 | Entry 3 |
| 10 | Entry 3 |
| 11 | Susceptible check |
| 12 | Entry 4 |
| 13 | Entry 4 |
| 14 | Parthenium |

*Observations* On the 30th DAS the total plant count was recorded. The first observation on virus incidence (number of plants infected/total number of plants) was recorded on the 40th DAS. The second observation on virus incidence was recorded on the 60th day of the crop. The third observation at seed setting stage was to record TSV disease symptoms. The symptom types considered for observation were mosaicing, leaf/stem necrosis, leaf yellowing, stunting, petiole necrosis and head necrosis.

Plants which remained symptom free until the seed setting stage were deemed TSV resistant plants.

*Typical growth conditions for sunflower plants* The typical weather conditions under which the sunflower plants of the invention were grown in the field are summarised in Figure 1. Weather data was collected at Mulani Wadgaon Farm, Aurangabad, India in 2008. The time of year in which the sunflower plants of the invention were typically grown was in one of 3 seasons: June to February, August to November or September to December. Sunflower plants containing the *TSV1* gene showed TSV resistance under variable conditions of rainfall and humidity throughout the year.

### EXAMPLE 2

### Introgression of TSV1 gene into elite Syngenta lines

The wild accession sunflower line containing the *TSV1* gene responsible for TSV resistance is *H. annuus* ANN2121 (obtained from USDA; accession number PI 586818; originally sourced from Montana, USA in September 1991). ANN2121 was crossed with elite Syngenta TSV susceptible sunflower line FR818 at Syngenta Seeds, Saint Sauveur, France. The resultant progeny was crossed with elite TSV susceptible Syngenta sunflower line RW666 to obtain a progeny of line (RW666/(FR818xANN2121 The line (RW666/(FR818xANN2121) was further crossed with elite Syngenta TSV susceptible sunflower line RW666RM to obtain a progeny of line (RW666RM//(RW666/(FR818xANN2121))). The progeny of line (RW666RM//(RW666/(FR818xANN2121))) was backcrossed 4 times to obtain an F5 generation plant. This F5 plant was designated 0GI1100. This line was tested along with parental and other lines under natural virus pressure at Syngenta Limited, Aurangabad, India in 2003 and found resistant for TSV (0GI1100 showed none of the TSV symptoms mosaicing, leaf/stem necrosis, leaf yellowing, stunting, petiole necrosis or head necrosis at the seed setting stage).

### EXAMPLE 3

### Virus testing of 0GI1100 alongside other Syngenta sunflower hybrids and lines in a polyhouse

*H. annuus* var. 0GI1100 was selfed to obtain pure seeds. This was ensured by covering the flower heads with cloth bags before opening and subsequent covering with nylon bags over the cloth bags with the onset of seed setting. F1 progeny having the most desirable agronomic characteristics such as good head size and good growth (VTR-2 and VTL-1) were tested for resistance to TSV by artificial inoculation in a polyhouse (see table 2). The susceptible checks used were PAC-36 and Morden lines. VTL1 and VTR2 were found to have clear resistance to TSV.

**Table 2 Comparing TSV resistance of 0GI1100 progeny against other Syngenta sunflower hybrids and lines in a polyhouse**

| **Pedigree/ Res. Code No.** | **Reaction on infected plants** | **Remarks** |
|---|---|---|
| VTR-2 | No symptoms | Resistant |
| VTL-1 | No symptoms | Resistant |
| 275AXOFT26173-14 | Paling and stunting | Not clear |
| 338AXOFT26173-14 | Paling and stunting | Not clear |
| LA-27XOFT26173-14 | Paling | Not clear |
| LA-39XOFT26173-14 | Paling | Not clear |
| LA-39X275R | Leaf necrosis | Susceptible |
| LA-39xSR-56 | Leaf necrosis | Susceptible |
| LA-27x278R | Leaf necrosis | Susceptible |
| 338Ax278R | Leaf necrosis | Susceptible |
| S-275 | Leaf necrosis | Susceptible |
| VSL-1 | Mosaic | Susceptible |
| VTR-1 | Leaf necrosis | Susceptible |
| VTA-3 | Leaf necrosis | Susceptible |
| VTA-4 | Leaf necrosis | Susceptible |
| VTA-1 | Leaf necrosis | Susceptible |
| VTA-2 | Leaf necrosis | Susceptible |
| PAC36xOFT26173-14(F2) | Leaf necrosis | Susceptible |
| 11604R | Leaf necrosis | Not clear |
| (39Bx275B)x275B (BC3F1) | Leaf necrosis | Susceptible |
| 8FT26173-14x39B (F1) | Leaf necrosis | Susceptible |
| (6D-1x39)-1-1-2 (F4) | Leaf necrosis | Susceptible |
| (6D-1x39)-1-1-3 (F4) | Leaf necrosis | Susceptible |
| 8FT26173-14 x 278R (F1) | Leaf necrosis | Susceptible |
| TO18890 | | Not clear |
| PAC-36 | Mosaic | Susceptible |
| Morden | Leaf necrosis | Susceptible |

### EXAMPLE 4

### Screening of inbred line VTR2 progeny for TSV resistance

The VTR2 line was selected for further analysis due to its desirable agronomic characteristics and strong resistance to TSV. VTR2 was selfed once as described above to provide F1 progeny which were then subject to further analysis. Of the F1 generation, progeny numbers 2, 30, 31, 33, 34 and 35 showed the best characteristics in terms of growth and TSV resistance. Seeds of these F1 progeny were then sown to obtain F2 progeny which were then screened for TSV resistance (see Table 3)

**Table 3 Screening of VTR2 progeny for TSV resistance.**

| **Entry Name*** | **% Infection** | **Symptom Type** | **Remarks** |
|---|---|---|---|
| VTR2-30-1 | 0 | No symptoms | Resistant |
| VTR2-30-2 | 0 | No symptoms | Resistant |
| VTR2-30-3 | 0 | No symptoms | Resistant |
| VTR2-30-4 | 0 | No symptoms | Resistant |
| VTR2-30-5 | 0 | No symptoms | Resistant |
| VTR2-30-6 | 0 | No symptoms | Resistant |
| VTR2-31-1 | 0 | No symptoms | Resistant |
| VTR2-31-2 | 0 | No symptoms | Resistant |
| VTR2-31-3 | 0 | No symptoms | Resistant |
| VTR2-31-4 | 0 | No symptoms | Resistant |
| VTR2-31-5 | 0 | No symptoms | Resistant |
| VTR2-31-6 | 0 | No symptoms | Resistant |
| VTR2-31-7 | 0 | No symptoms | Resistant |
| VTR2-31-8 | 0 | No symptoms | Resistant |
| VTR2-31-9 | 0 | No symptoms | Resistant |
| VTR2-31-10 | 0 | No symptoms | Resistant |
| VTR2-32-1 | 0 | No symptoms | Resistant |
| VTR2-33-1 | 0 | No symptoms | Resistant |
| VTR2-35-1 | 0 | No symptoms | Resistant |
| VTR2-35-2 | 0 | No symptoms | Resistant |
| VTR2-15-1 | 0 | No symptoms | Resistant |
| VTR2-15-2 | 0 | No symptoms | Resistant |
| VTR2-15-3 | 0 | No symptoms | Resistant |
| VTR2-15-4 | 0 | No symptoms | Resistant |
| VTR2-05-1 | 0 | No symptoms | Resistant |
| Susceptible check | 100 | Mosaic | Susceptible |

From the table above, it can be seen that the F2 progeny of VTR2 were resistant to TSV.

After many selections, the three best progenies VTR2-15, VTR2-30 and VTR2-33 were chosen for hybrid development.

The selected progeny lines had the following characteristics:
1) VTR2-15 shows early maturity, medium tall, full branching, dark green broad leaves with coarse serration, golden yellow flower, convex head, lateral head to central head with curved stem and black non striped seed;
2) VTR2-30 shows medium maturity, tall plant height, full branching, dark green broad leaves with coarse serration, light yellow flower, flat head, lateral head to central head with curved stem and black non striped seeds;
3) VTR2-33 shows late maturity, very tall plant height, full branching, dark green broad leaf with coarse serration, golden yellow flower, semi- convex head, lateral head to central head with curved stem and black non striped seed.

During flowering, pollen of the VTR2 progeny were used in a crossing block as restorer with different CMS lines for the development of new TSV resistant hybrid sunflower plants.

### EXAMPLE 5

### Evaluation of TSV resistant hybrids at different locations during the rainy season

Stage 5 (set 1 and 2) TSV resistant hybrids from example 4 were tested along with susceptible commercial hybrid PAC-8699 at Kadappa, Andhra Pradesh, India. Plot size was 6x2.4 (4 rows each of 6m length). Spacing was 60x30cm. Fertiliser used was 60-90-40 (N-P-K) kg/ha. Oil content was determined by University of Agricultural Sciences, Raichur, Main Agricultural Research Station, Raichur - 584 102, Karnatka. Days taken to reach maturity from the date of sowing are also shown.

**Table 4: Performance of sunflower hybrid Stage-5 at Kadappa, Andhra Pradesh during rainy season.**

| **Hybrid** | **% Infection at 60 DAS** | **Days to 50 % Flowering** | **Days to Maturity** | **Seed Yield (kg/ha)** | **Volume Weight (gm/cc)** | **Oil Content (%)** |
|---|---|---|---|---|---|---|
| S 275 | 0 | 59 | 95 | 856 | 42.50 | 41 |
| S275V | 0 | 63 | 97 | 678 | 36.00 | 41 |
| Nk Armony V | 0 | 62 | 98 | 1094 | 35.00 | 41 |
| S 293 | 0 | 54 | 85 | 980 | 40.00 | 41 |
| PAC 8699 | 35 | 68 | 118 | 603 | 39.00 | 38 |

### EXAMPLE 6

### Allelic test for TSV1 gene

An allelic test is carried out as follows between a plant of a TSV resistant male breeding line of *H. annuus* (0GI1100) comprising a monogenic dominant homozygous *TSV1* gene (parent 1) and a plant to be tested of a female breeding line of *H. annuus* which is also TSV resistant when exposed to the same environmental conditions (parent 2).

For the development of an F1 population, parents 1 and 2 (P1 and P2) are sown at Syngenta India Limited, Aurangabad, India. Plants which are 2 months old are then crossed, and F1 seed is harvested 1 month later. For F2 and testcross development, approximately 40 F1 seedlings are selfed or testcrossed to obtain F2 and BC1F1 seed. Approximately 50 P1 plants, 50 F1 plants, 400 F2 plants, 250 BC1F1 plants and 50 P2 plants are then screened for TSV resistance as herein described.

When the plant to be tested has *TSV1* as a sole source of resistance, all of the F1 progeny are TSV resistant. When the plant to be tested is homozygous for the *TSV1* gene then all of the F2 plants are also TSV resistant. However, when the plant to be tested is heterozygous for the *TSV1* gene, then 50% of the F1 progeny produce F2 which are 100% TSV resistant whereas the other 50% of the F1 progeny produce F2 which are 3:1 resistant:susceptible.

A different outcome is obtained when the plant to be tested has a dominant gene, other than *TSV1* (and not linked to *TSV1*), which provides the sole source of TSV resistance. Again, all of the F1 progeny are TSV resistant. However, when the plant to be tested is homozygous for a gene providing resistance other than *TSV1,* the F2 progeny segregate 15:1 resistant:susceptible. When the plant to be tested is heterozygous for a gene providing resistance other than *TSV1,* then 50% of the F1 progeny produce F2 which are 15:1 resistant:susceptible whereas the other 50% of the F1 progeny produce F2 which are 3:1 resistant:susceptible.

In practice there can sometimes be a slight deviation between the above observed and expected ratios, however this should not be substantial when the allelic test is performed under consistent environmental conditions.

## Claims

1. A cultivated plant comprising a gene, *TSV1,* conferring resistance to tobacco streak virus disease.

2. A cultivated plant according to claim 1, wherein the plant is a sunflower plant selected from the group *Helianthus annuus*, *Helianthus tuberosus.*

3. A cultivated plant according to claim 1 or 2, wherein the gene is derivable from a sunflower plant selected from the group *Helianthus annuus, Helianthus tuberosus.*

4. A cultivated plant according to claim 2 or 3, wherein the sunflower plant which the gene is derivable from is cultivated inbred line *H. annuus* var 0GI1100, obtainable from seed represented by NCIMB accession number NCIMB 41748.

5. A cultivated plant according to any one of the preceding claims, in which the gene can be introduced into a sunflower plant.

6. A cultivated plant according to any one of the preceding claims in which said gene can be detected by crossing the cultivated plant with a plant of line *H*. *annuus* var 0GI1100, representative seed of which has been deposited under accession number NCIMB 41748 wherein 100% of F1 plants resulting from said cross are resistant to TSV and a) 100% of said F1 plants produce 100% of F2 progeny plants resistant to TSV, when said sunflower plant is homozygous for the *TSV1* gene; or b) 50% of said F1 plants produce 100% of F2 progeny plants resistant to TSV and 50% of said F1 plants a 3:1 ratio of F2 progeny plants resistant to TSV to F2 progeny plants susceptible to TSV, when said sunflower is heterozygous for the *TSV1* gene.

7. A method of producing sunflower oil and/or meal comprising the steps:
a) growing and harvesting a cultivated sunflower plant comprising the *TSV1* gene according to claims 1 to 6;
b) processing seed obtained from the harvested sunflower plant to produce oil and/or meal.

8. A method according to claim 7, wherein the harvested sunflower plant of step a) does not exhibit any of the following disease symptoms: mosaicing, general necrosis, stem necrosis, yellowing, stunting and/or head necrosis at the seed setting stage.

9. A method according to claim 7 and 8, wherein the sunflower plant of step a) is hybrid line S-293, obtainable from seed represented by NCIMB accession number NCIMB 41747, or *H. annuus* var. 0GI1100, obtainable from seed represented by NCIMB accession number NCIMB 41748.

10. A method of producing sunflower oil and/or meal comprising the step of processing seed obtained from a cultivated sunflower plant comprising the *TSV1* gene according to any one of claims 1-6 to produce oil and/or meal.

11. A method according to claim 10, wherein the cultivated sunflower plant does not exhibit any of the following disease symptoms: mosaicing, general necrosis, stem necrosis, yellowing, stunting and/or head necrosis at the seed setting stage.

12. A method according to any one of claims 10 and 11, wherein the cultivated sunflower plant is hybrid line S-293, obtainable from seed represented by NCIMB accession number NCIMB 41747, or *H. annuus* var. 0GI1100, obtainable from seed represented by NCIMB accession number NCIMB 41748.

13. A method of producing seed from a tobacco streak virus resistant cultivated sunflower plant containing the gene of any one of claims 1 to 6 comprising obtaining seeds from the cultivated sunflower plant that has been grown and harvested.

14. Oil and/or meal obtained by a method of any one of claims 7 to 13.

15. Oil and/or meal obtained from a cultivated sunflower plant comprising the *TSV1* gene according to any one of claims 1-6, or a fragment thereof.

16. Oil and/or meal containing the *TSV1* gene according to any one of claims 1-6, or a fragment thereof.

17. Food product comprising oil and/or meal of claims 14 to 16.
